Europäisches Patentamt

(19) European Patent Office

Office européen des brevets

(11) Numéro de publication : **0 329 032 B1**

(12) **FASCICULE DE BREVET EUROPEEN**

(45) Date de publication du fascicule du brevet :
08.01.92 Bulletin 92/02

(51) Int. Cl.⁵ : **A61K 7/06**, A61K 7/00, **A61K 7/42**

(21) Numéro de dépôt : **89102334.3**

(22) Date de dépôt : **10.02.89**

(54) Utilisation de l'acide 4-(2-oxo-3-bornylidèneméthyl) benzène sulfonique ou ses sels pour préserver les propriétés mécaniques des cheveux des agressions atmosphériques.

(30) Priorité : **11.02.88 LU 87131**

(43) Date de publication de la demande :
**23.08.89 Bulletin 89/34**

(45) Mention de la délivrance du brevet :
**08.01.92 Bulletin 92/02**

(84) Etats contractants désignés :
**AT BE CH DE ES FR GB GR IT LI NL SE**

(56) Documents cités :
**FR-A- 2 282 426**
**FR-A- 2 509 989**
**FR-A- 2 548 018**
**GB-A- 2 185 019**
**GB-A- 2 195 243**

(73) Titulaire : **L'OREAL**
**14, Rue Royale**
**F-75008 Paris (FR)**

(72) Inventeur : **Grollier, Jean-François**
**16Bis Boulevard Morland**
**F-75004 Paris (FR)**
Inventeur : **Dubief, Claude**
**9, Rue Edmond Rostand**
**F-78150 Le Chesnay (FR)**
Inventeur : **Fourcadier, Chantal**
**6 Passage des Entrepreneurs**
**F-75015 Paris (FR)**

(74) Mandataire : **Casalonga, Axel et al**
**BUREAU D.A. CASALONGA - JOSSE**
**Morassistrasse 8**
**W-8000 München 5 (DE)**

## Description

Utilisation de l'acide 4-(2-oxo-3-bornylidèneméthyl) benzène sulfonique ou ses sels pour la protection des cheveux contre les agressions atmosphériques, et en particulier contre la lumière, et procédé de protection des cheveux utilisant ce composé.

La présente invention a pour objet l'utilisation de l'acide 4-(2-oxo-3-bornylidèneméthyl) benzène sulfonique et de ses sels en tant qu'agents de protection de la kératine des cheveux contre les agressions atmosphériques, et en particulier contre la lumière, et un procédé de protection des cheveux contre les agressions atmosphériques, et en particulier contre la lumière.

On sait depuis longtemps que la lumière agresse la kératine des cheveux. De nombreuses publications divulguent que la lumière naturelle détruit certains aminoacides des cheveux et qu'en altérant la fibre capillaire, elle en diminue les propriétés mécaniques ; par diminution des propriétés mécaniques, on entend principalement la diminution du palier à 15% d'extension.

Le palier à 15% d'extension est le poids qu'il faut appliquer à un cheveu mouillé d'une longueur donnée pour l'allonger de 15%. Plus le poids est élevé, plus le cheveu est élastique et résistant.

Pour lutter contre l'agression de la kératine des cheveux par la lumière, on a déjà proposé d'utiliser des substances susceptibles de filtrer les radiations lumineuses. En particulier, on a essayé des agents filtrants bien connus de la technique, tels que des dérivés de la benzophénone, par exemple la 2-hydroxy-4-méthoxybenzophénone et la 2,2'-dihydroxy-4,4'-diméthoxy-5,5'-disulfobenzophénone disodique ou encore des dérivés du dibenzoylméthane, par exemple le 4-tert-butyl-4'-méthoxydibenzoylméthane ou l'acide p-aminobenzoïque et ses dérivés tels que le p-diméthylaminobenzoate de 2-éthylhexyle, le tosylate de (4'-diméthylaminobenzoyloxyéthyl), diméthyl, alkyl ($C_{16}$-$C_{18}$) ammonium (ESCALOL 537 Q), des cinnamates tels que le p-méthoxycinnamate de 2-éthylhexyle ou encore l'acide 2-phénylbenzimidazole-5-sulfonique.

On a également proposé d'utiliser un filtre hydrosoluble comme le méthylsulfate de 4-(2-oxo-3-bornylidèneméthyl)phényltriméthylammonium (FR-A-2509989) ou l'acide 3-[4-(5-sulfobenzimidazol-2-yl)benzylidène] bornanone-2 (GB-A-2185019).

Cependant, ces substances filtrantes ne se sont pas révélées efficaces pour préserver les propriétés mécaniques des cheveux, à savoir leur élasticité, contre les effets néfastes de la lumière.

Il est, au contraire, apparu que leur présence dans certaines compositions cosmétiques pouvait même accentuer la dégradation des propriétés mécaniques, notamment la diminution du palier à 15% d'extension.

La demanderesse a maintenant découvert, de façon surprenante, que l'acide 4-(2-oxo-3-bornylidèneméthyl) benzène sulfonique et ses sels pouvaient préserver, les propriétés mécaniques des cheveux de la dégradation par la lumière. Cette propriété a pu être mise en évidence par exposition en lumière naturelle (milieu ensoleillé) et en lumière artificielle (émetteur au xénon d'un appareil de vieillissement accéléré du type SUNTEST HANAU).

La présente invention a donc pour objet l'utilisation de l'acide 4-(2-oxo-3-bornylidèneméthyl) benzène sulfonique et de ses sels en tant qu'agents de protection des propriétés mécaniques des cheveux, et essentiellement du palier à 15% d'extension au mouillé, contre la dégradation provoquée par les agressions atmosphériques, et en particulier par la lumière.

Par sels de l'acide sulfonique précité, on entend les sels métalliques et plus particulièrement alcalins, alcalino-terreux, ou les sels d'ammonium et d'amines.

Selon la présente invention, on utilise l'acide sulfonique précité ou ses sels pour préserver les propriétés mécaniques des cheveux des agressions de la lumière, en des quantités au moins égales à 0,3% en poids d'acide libre (avant neutralisation éventuelle), et de préférence comprises entre 0,4% et 9% en poids, dans un support aqueux, alcoolique ou hydroalcoolique, cosmétiquement acceptable. La quantité minimale de 0,3% correspond à une concentration molaire pour 100 g de composition d'environ 0,9 millimole d'acide qui peut être utilisé sous forme libre ou salifié par un hydroxyde métallique, l'ammoniaque ou une amine.

L'acide sulfonique ou ses sels selon l'invention peuvent être utilisés pour protéger les cheveux naturels ou sensibilisés. On entend par "cheveux sensibilisés", des cheveux ayant subi un traitement de permanente, de coloration ou de décoloration.

Les compositions cosmétiques pour cheveux, utilisées conformément à l'invention, pour les protéger contre la dégradation par la lumière et contenant à titre de composé actif l'acide 4-(2-oxo-3-bornylidèneméthyl) benzène sulfonique et ses sels, peuvent se présenter sous forme de solutions aqueuses, alcooliques ou hydroalcooliques (l'alcool étant le plus souvent un alcanol inférieur tel que l'éthanol ou l'isopropanol), épaissies ou non, de gels, de mousses aérosols ou de sprays et contenir les adjuvants habituellement utilisés dans les compositions capillaires et adaptés à l'application envisagée.

Ces compositions peuvent être ou non suivies d'un rinçage et peuvent constituer des shampooings, des après-shampooings, des produits à rincer à appliquer avant ou après shampooing, avant ou après coloration ou décoloration, avant ou après permanente ou défrisage, des compositions non rincées telles que des lotions, gels, sprays ou mousses pour la mise en plis, pour le brushing, des laques ou sprays pour le maintien de la coiffure et des compositions restructurantes.

Lorsque les compositions cosmétiques utilisées selon l'invention constituent des compositions non suivies d'un rinçage, l'acide 4-(2-oxo-3-bornylidènemethyl) benzène sulfonique ou ses sels, en tant qu'agent actif préservant les propriétés mécaniques des cheveux contre la lumière, est présent à raison de 0,3 à 5% en poids par rapport au poids total de la composition, et de préférence à raison de 0,4 à 3,5% en poids d'acide libre.

Lorsque les compositions cosmétiques utilisées selon l'invention constituent des compositions suivies d'un rinçage, l'acide ou ses sels est présent à raison de 0,5 à 9% en poids, et de préférence de 0,5 à 6% en poids d'acide libre, par rapport au poids total de la composition.

Les compositions cosmétiques pour cheveux selon l'invention ont un pH compris entre 2 et 9, et de préférence entre 4 et 8.

Les compositions cosmétiques utilisées selon l'invention peuvent également renfermer des agents cosmétiques bien connus dans la technique sous réserve qu'ils n'altèrent pas eux-mêmes les propriétés mécaniques de la kératine des cheveux.

Les adjuvants ou agents cosmétiques généralement présents dans les compositions cosmétiques utilisées selon l'invention sont par exemple des agents de surface cationiques, anioniques, amphotères, non-ioniques ou leurs mélanges, des épaississants, des polymères anioniques, cationiques, non-ioniques, amphotères ou leurs mélanges, des adoucissants, des conservateurs, des agents de moussage, des stabilisateurs de mousse, des électrolytes, des agents de régulation de pH, des agents anti-gras, des agents séquestrants, des parfums, des colorants, des propulseurs, des solvants organiques.

Les agents tensio-actifs cationiques, anioniques, non-ioniques, amphotères ou leurs mélanges sont généralement utilisés dans des proportions de 0,1 à 50% en poids, et de préférence de 0,5 à 30% en poids par rapport au poids total de la composition.

Lorsque les compositions cosmétiques pour cheveux utilisées selon l'invention constituent des shampooings, ceux-ci sont essentiellement caractérisés par le fait qu'ils contiennent, outre l'acide sulfonique ou ses sels ci-dessus défini, au moins un agent de surface anionique, non-ionique, cationique, amphotère ou un mélange de tels agents de surface, en milieu aqueux. Les shampooings peuvent également contenir différents adjuvants tels que des colorants, des conservateurs, des agents épaississants, des agents stabilisateurs de mousse, des synergistes, des agents adoucissants, des électrolytes, des séquestrants, une ou plusieurs résines cosmétiques, des parfums, des essences naturelles ainsi que tout autre adjuvant utilisé habituellement dans un shampooing. Dans ces shampooings, la concentration d'agent de surface est généralement comprise entre 2 et 50% en poids. Leur pH est généralement compris entre 3 et 9.

Lorsque les compositions utilisées selon l'invention constituent des compositions non rincées — lotion, gel, mousse, spray ou laque pour le brushing, pour la mise en plis, pour coiffer ou traiter la chevelure — elles comprennent généralement, dans un milieu aqueux, alcoolique ou hydroalcoolique, outre l'acide sulfonique ou ses sels défini ci-dessus, au moins un polymère cationique, anionique, non-ionique, amphotère ou un mélange de tels polymères, dans des quantités comprises généralement entre 0,1 et 10%, et de préférence entre 0,1 et 3% en poids, et éventuellement des agents anti-moussants.

Lorsque les compositions capillaires selon l'invention constituent des lotions rincées, appelées également "rinse", elles sont appliquées avant ou après coloration ou décoloration, avant ou après permanente, avant ou après shampooing ou entre deux temps de shampooing, puis rincées après un temps de pose.

Ces compositions peuvent être des solutions aqueuses ou hydroalcooliques comprenant éventuellement des tensio-actifs ; elles peuvent être aussi des gels. Ces compositions peuvent également être pressurisées en aérosol sous forme de sprays ou mousses.

Dans ces compositions rincées, la concentration en agents tensio-actifs peut varier entre 0,1 et 10%, et de préférence entre 0,5 et 7% en poids. Elles peuvent également contenir des polymères non-ioniques, cationiques, anioniques, amphotères ou leurs mélanges.

Quand les compositions capillaires se présentent sous forme de gels, à rincer ou non, elles contiennent des épaississants en présence ou non de solvants.

Les épaississants peuvent être l'alginate de sodium, la gomme arabique ou la gomme de xanthane ou des dérivés cellulosiques tels que la méthyl cellulose, l'hydroxyméthyl cellulose, l'hydroxyéthyl cellulose, l'hydroxypropyl cellulose, la carboxyméthyl cellulose ou des polymères carboxyliques tels que les "Carbopol". On peut également obtenir un épaississement des lotions par mélange de polyéthylène glycol et de stéarate ou distéarate de polyéthylène glycol ou par un mélange d'esters phosphoriques et d'amides. La concentration en épaississant peut varier de 0,1 à 30%, et de préférence de 0,2 à 15% en poids, par rapport au poids total de la composition.

On peut utiliser également un agent épaississant qui résulte de l'interaction ionique d'un polymère cationique constitué par un copolymère de cellulose ou d'un dérivé de cellulose greffés par un sel de monomère hydrosoluble d'ammonium quaternaire (vendus sous les dénominations "CELQUAT H 100" ou "CELQUAT L 200") et d'un polymère anionique carboxylique ayant une viscosité capillaire absolue dans le diméthylformamide ou le méthanol à une concentration de 5% et à 30°C, inférieure ou égale à $30.10^{-3}$ Pa $\cdot$ s.

Cet épaississant est décrit dans la demande de brevet FR 2598611.

Quand les compositions capillaires se présentent sous forme de mousses, à rincer ou non, elles-contiennent un agent de moussage en milieu aqueux ou hydroalcoolique, en présence d'un gaz propulseur.

Comme agent de moussage, on peut utiliser des tensio-actifs anioniques, non-ioniques, cationiques, amphotères ou leurs mélanges, des polymères non-ioniques, anioniques, cationiques ou leurs mélanges, de l'alcool polyvinylique issu de polyacétate de vinyle hydrolysé dont le taux d'hydrolyse est égal ou inférieur à 97% comme décrit dans la demande de brevet FR 2598613.

Pour former une mousse, on préfère utiliser une association de polymère cationique et de polymère anionique, au moins l'un des deux polymères étant moussant en solution aqueuse. De telles associations sont décrites dans le brevet FR 2505348. Les gaz propulseurs utilisés pour pressuriser ces compositions destinées à former des mousses sont présents dans des proportions ne dépassant pas 25% et de préférence 15% par rapport au poids total de la composition. On peut utiliser à titre de gaz propulseur le gaz carbonique, l'azote, le protoxyde d'azote, les hydrocarbures volatils tels que le butane, l'isobutane, le propane et leurs mélanges, les hydrocarbures chlorés et/ou fluorés non hydrolysables tels que ceux vendus sous les dénominations "FREON" ou "DYMEL" par la Société DU PONT de NEMOURS.

Lorsque la composition se présente sous forme de spray ou de laque, elle renferme dans un milieu alcoolique ou hydroalcoolique, une résine filmogène en présence éventuellement d'un gaz propulseur. On utilise de préférence, comme résine filmogène, un polymère anionique contenant des unités d'acides acrylique ou méthacrylique, d'acide crotonique ou d'acides α,β-dicarboxyliques insaturés.

Les agents propulseurs utilisés dans les formulations sous forme de laques peuvent être choisis parmi les hydrocarbures volatils tels que le n-butane, le propane, l'isobutane ou leurs mélanges ou un mélange de ces hydrocarbures avec des hydrocarbures chlorés et/ou fluorés tels que les composés vendus sous la dénomination de "FREON" ou "DYMEL" par la Société DU PONT de NEMOURS et plus particulièrement les hydrocarbures fluorochlorés tels que le monofluorotrichlorométhane, le difluorodichlorométhane, le tétrafluorodichloroéthane ou les mélanges de ces derniers.

Ils peuvent également être choisis parmi les hydrocarbures chlorés et/ou fluorés susdécrits et leurs mélanges, le diméthyléther, le gaz carbonique ou le protoxyde d'azote.

La phase propulsive, dans ces compositions de laque, représente 30 à 80% du poids total de la composition pressurisée.

Quand les compositions capillaires de l'invention constituent des lotions restructurantes, elles contiennent des produits renforçant la chaîne kératinique des cheveux. A cette classe de produits appartiennent les dérivés méthylolés tels que ceux décrits dans les brevets français n° 1527085 et 1519979.

La présente invention vise également un procédé de protection de la kératine des cheveux contre les agressions atmosphériques, et en particulier contre la lumière, consistant à appliquer sur les cheveux une quantité efficace d'acide 4-(2-oxo-3-bornylidèneméthyl) benzène sulfonique ou de ses sels, dans un support aqueux, alcoolique ou hydroalcoolique, cosmétiquement acceptable.

Les exemples suivants illustrent l'invention sans pour autant la limiter.

EXEMPLE 1

On prépare une composition à rincer protectrice des cheveux :

- Acide 4-(2-oxo-3-bornylidèneméthyl) benzène
sulfonique     5   g
- Alkyl ($C_{12}$-$C_{14}$) éther sulfate de sodium
oxyéthyléné à 2,2 moles d'oxyde d'éthylène en
solution aqueuse à 25% de matière active (MA)    2   g MA
- Hydroxyéthyl cellulose vendue par la société
HERCULES sous la dénomination "NATROSOL 250 HHR"   1   g
- Triéthanolamine     qs pH : 7
- Colorant, parfum, conservateur     qs
- Eau     qsp   100   g

Cette composition est appliquée sur des cheveux décolorés, puis rincée. Les cheveux sont alors exposés 120 heures au Suntest à l'aide d'un appareil "SUNTEST HANAU". Cet appareil est constitué d'un émetteur au Xénon et d'un système de filtres produisant un rayonnement correspondant dans une très large mesure au rayonnement solaire. Le rayonnement énergétique est de 585 W/m² environ dans le domaine de longueurs d'onde comprises entre 300 et 830 nm (rayonnement global).

Le traitement avec cette composition permet d'améliorer la valeur moyenne du palier à 15% d'extension au mouillé de manière très significative par rapport à des cheveux de même nature traités de façon identique mais avec une composition à rincer ne renfermant pas d'acide 4-(2-oxo-3-bornylidèneméthyl) benzène sulfonique.

## EXEMPLE 2

On prépare une mousse de coiffage protectrice des cheveux de composition suivante :

- Acide 4-(2-oxo-3-bornylidèneméthyl) benzène
sulfonique     1,5   g
- Copolymère vinylméthyléther/anhydride maléique
monoestérifié avec le butanol, vendu à 50% de
matière active (MA) dans l'éthanol sous la
dénomination "GANTREZ ES 425" par la Société
GENERAL ANILIN     0,6   g MA
- Hydroxyéthylcellulose greffée par du chlorure
de diallyldiméthyl ammonium, vendue par la
société NATIONAL STARCH sous la dénomination
"CELQUAT L 200"     0,5   g
- Polymère cationique siliconé vendu par la
société UNION CARBIDE sous la dénomination
"UCAR SILICONE ALE 56" en solution aqueuse
à 35% de matière active (MA)     0,2   g MA
- Alcool éthylique     qs : 10°
- 2-amino-2-méthyl-propan-1-ol     qs   pH : 7,5
- Parfum, colorant, conservateur     qs
- Eau     qsp   100   g

On conditionne la composition ci-dessus dans un dispositif aérosol :

```
    - Composition              90 g
    - Fréons 12/114 (57/43)    10 g
                    en poids
                    _____

            Total              100 g
```

Fréon 12  = difluorodichlorométhane

Fréon 114 = 1,2-dichlorotétrafluoroéthane

Après trois applications de cette mousse sur des cheveux décolorés et sans rinçage consécutif, on expose les cheveux 180 heures au Suntest, comme décrit à l'exemple 1.

Par rapport à des cheveux de même nature et traités de façon identique mais avec une mousse sans acide 4-(2-oxo-3-bornylidèneméthyl) benzène sulfonique, on constate une amélioration appréciable de la valeur moyenne du palier à 15% d'extension au mouillé.

EXEMPLE 3

On prépare un spray de coiffage protecteur de la chevelure de composition suivante :

```
    - Acide 4-(2-oxo-3-bornylidèneméthyl) benzène
      sulfonique                                    1    g
    - Terpolymère acide crotonique/4-tert.-butyl
      benzoate de vinyle/acétate de vinyle (10/25/65)
      préparé selon le brevet français n° 2 439 798
      (exemple 19)                                  6    g
    - 2-amino-2-méthyl-propan-1-ol     qs  pour neutralisation
    - Parfum, conservateur              qs
    - Alcool éthylique                  qsp   100    g
```

On conditionne la composition ci-dessus dans un flacon pompe.

Des cheveux décolorés sont traités par une application de ce spray et sans être rincés, sont ensuite exposés 120 heures au Suntest dans les conditions décrites à l'exemple 1. Comparativement à des cheveux de même nature et traités de façon analogue mais avec un spray ne renfermant pas d'acide 4-(2-oxo-3-bornylidèneméthyl) benzène sulfonique, on enregistre une amélioration significative de la valeur moyenne du palier à 15% d'extension au mouillé.

EXEMPLE 4

On prépare un gel de coiffage protecteur de la chevelure de composition suivante :

- Acide 4-(2-oxo-3-bornylidèneméthyl) benzène
  sulfonique                                           0,4  g
- Acide polyacrylique réticulé PM : 4 millions
  vendu sous la dénomination "CARBOPOL 940" par
  la Société GOODRICH                                   0,5  g
- 2-amino 2-méthyl propan-1-ol          qs    pH        7,5
- Parfum, colorant, conservateur         qs
- Alcool éthylique                       qs      10°
- Eau                                    qsp      100  g

Des cheveux décolorés sont traités par une application de ce gel et sans être rincés, sont ensuite exposés 120 heures au Suntest dans les conditions décrites à l'exemple 1. Comparativement à des cheveux de même nature et traités de façon analogue mais avec un gel ne renfermant pas d'acide 4-(2-oxo-3-bornylidène méthyl) benzène sulfonique, on enregistre une amélioration appréciable de la valeur moyenne du palier à 15% d'extension au mouillé.

EXEMPLE 5

On prépare un shampooing protecteur des cheveux de composition suivante :

- Alkyl ($C_{12}$-$C_{14}$) éther sulfate de sodium oxyéthyléné
  à 2,2 moles d'oxyde d'éthylène en solution aqueuse
  à 25% MA                                                  3 g MA
- α-oléfine ($C_{14}$-$_{16}$) sulfonate de sodium vendu en
  solution aqueuse à 38% MA par la Société AKZO sous
  la dénomination "ELFAN OS46"                              3 g MA
- Tensio-actif dénommé "COCOAMPHOCARBOXY-GLYCINATE"
  (CTFA 3e édition 1982), vendu sous la dénomination
  "MIRANOL CLM conc." par la Société MIRANOL, en
  solution aqueuse à 38% MA                                 4 g MA
- Distéarate de glycol                                      1 g
- Diéthanolamide d'acide de coprah vendu sous la
  dénomination "COMPERLAN KD" par la Société HENKEL         2 g
- Gomme de xanthane vendue par la Société KELCO
  sous la dénomination "KELTROL T"                          0,6 g
- Acide 4-(2-oxo-3-bornylidène méthyl)benzène
  sulfonique                                                1 g
- Conservateur(s), parfum, colorant,        qs
- Eau                                                qsp    100 g
- HCl                               qs          pH : 5

Après quelques applications de ce shampooing, on constate une amélioration appréciable de la valeur moyenne du palier à 15% d'extension au mouillé.

7

## Revendications

**Revendications pour les Etats Contractants suivants : AT, BE, CH, DE, FR, GB, GR IT, LI, NL, SE**

1. Utilisation de l'acide 4-(2-oxo-3-bornylidèneméthyl) benzène sulfonique ou de ses sels pour préserver les propriétés mécaniques des cheveux de la dégradation par les agressions atmosphériques, et en particulier par la lumière.

2. Utilisation selon la revendication 1 de l'acide 4-(2-oxo-3-bornylidèneméthyl) benzène sulfonique ou de ses sels en une quantité au moins égale à 0,3% en poids d'acide libre, dans un support aqueux, alcoolique ou hydroalcoolique, cosmétiquement acceptable.

3. Utilisation selon la revendication 1 de l'acide 4-(2-oxo-3-bornylidèneméthyl) benzène sulfonique ou de ses sels en des quantités comprises entre 0,4 et 9% en poids d'acide libre, dans un support aqueux, alcoolique ou hydroalcoolique, cosmétiquement acceptable.

4. Utilisation selon l'une quelconque des revendications 1 à 3, des sels alcalins, alcalino-terreux, d'ammonium ou d'amines de l'acide 4-(2-oxo-3-bornylidèneméthyl) benzène sulfonique.

5. Utilisation selon l'une quelconque des revendications 1 à 4 de l'acide 4-(2-oxo-3-bornylidèneméthyl)benzène sulfonique ou de ses sels dans des compositions cosmétiques pour cheveux se présentant sous forme de solutions aqueuses, alcooliques ou hydroalcooliques, épaissies ou non, de gels, de mousses aérosols ou de sprays.

6. Utilisation selon la revendication 5 de l'acide 4-(2-oxo-3-bornylidèneméthyl) benzène sulfonique ou de ses sels dans des compositions cosmétiques pour cheveux contenant en outre au moins un adjuvant cosmétique choisi parmi les agents de surface cationiques, anioniques, amphotères, non-ioniques ou leurs mélanges, les épaississants, les polymères anioniques, cationiques, non-ioniques, amphotères ou leurs mélanges, les adoucissants, les conservateurs, les agents de moussage, les stabilisateurs de mousse, les électrolytes, les agents de régulation du pH, les agents anti-gras, les agents séquestrants, les parfums, les colorants, les propulseurs et les solvants organiques.

7. Utilisation selon l'une quelconque des revendications 1 à 6 de l'acide 4-(2-oxo-3-bornylidèneméthyl) benzène sulfonique ou de ses sels dans des compositions cosmétiques rincées, aqueuses, alcooliques ou hydroalcooliques, en des quantités comprises entre 0,5 et 9% en poids, et de préférence entre 0,5 et 6% en poids d'acide libre, par rapport au poids total de la composition.

8. Utilisation selon l'une quelconque des revendications 1 à 6 de l'acide 4-(2-oxo-3-bornylidèneméthyl)benzène sulfonique ou de ses sels dans des compositions cosmétiques non rincées, aqueuses, alcooliques ou hydroalcooliques, en des quantités comprises entre 0,3 et 5% en poids, et de préférence entre 0,4 et 3,5% en poids d'acide libre.

9. Utilisation selon l'une quelconque des revendications 1 à 7 de l'acide 4-(2-oxo-3-bornylidèneméthyl) benzène sulfonique ou de ses sels dans des shampooings contenant, outre l'acide sulfonique ou ses sels, au moins un agent de surface anionique, non-ionique, cationique, amphotère ou leurs mélanges, dans des proportions comprises entre 2 et 50% en poids, en milieu aqueux.

10. Utilisation selon l'une quelconque des revendications 1 à 8 de l'acide 4-(2-oxo-3-bornylidèneméthyl) benzène sulfonique ou de ses sels dans des compositions cosmétiques sous forme de mousses, à rincer ou non, contenant en milieu aqueux ou hydroalcoolique, en présence d'un gaz propulseur, un agent de moussage choisi parmi les tensio-actifs anioniques, non-ioniques, cationiques, amphotères ou leurs mélanges, les polymères non-ioniques, anioniques, cationiques ou leurs mélanges et l'alcool polyvinylique issu de polyacétate de vinyle hydrolysé dont le taux d'hydrolyse est égal ou inférieur à 97%.

11. Utilisation selon la revendication 10, de l'acide 4-(2-oxo-3-bornylidèneméthyl) benzène sulfonique ou de ses sels, dans une composition cosmétique sous forme de mousse, à rincer ou non, contenant une association d'un polymère cationique et d'un polymère anionique en milieu aqueux ou hydroalcoolique, au moins l'un des deux polymères étant moussant en solution aqueuse, en présence d'un gaz propulseur.

12. Utilisation selon l'une quelconque des revendications 1 à 8 de l'acide 4-(2-oxo-3-bornylidèneméthyl) benzène sulfonique ou de ses sels dans un spray, contenant dans un milieu alcoolique ou hydroalcoolique, éventuellement en présence d'un gaz propulseur, une résine filmogène constituée par un polymère anionique contenant des unités d'acides acrylique ou méthacrylique, d'acide crotonique ou d'acides $\alpha,\beta$-dicarboxyliques insaturés.

13. Procédé de protection de la kératine des cheveux contre les agressions atmosphériques, et en particulier contre la lumière, caractérisé par le fait qu'il consiste à appliquer sur les cheveux, une quantité efficace d'acide 4-(2-oxo-3-bornylidèneméthyl) benzène sulfonique ou de ses sels, dans un support aqueux, alcoolique ou hydroalcoolique, cosmétiquement acceptable.

14. Procédé selon la revendication 13, caractérisé par le fait qu'il consiste à appliquer sur les cheveux une composition contenant au moins 0,3% en poids d'acide 4-(2-oxo-3-bornylidènemethyl) benzène sulfonique, dans un support aqueux, alcoolique ou hydroalcoolique, cosmétiquement acceptable.

## Revendications pour l'Etat Contractant suivant : ES

1. Utilisation de l'acide 4-(2-oxo-3-bornylidènemethyl) benzène sulfonique ou de ses sels pour préserver les propriétés mécaniques des cheveux de la dégradation par les agressions atmosphériques, et en particulier par la lumière.

2. Utilisation selon la revendication 1 de l'acide 4-(2-oxo-3-bornylidènemethyl) benzène sulfonique ou de ses sels en une quantité au moins égale à 0,3% en poids d'acide libre, dans un support aqueux, alcoolique ou hydroalcoolique, cosmétiquement acceptable.

3. Utilisation selon la revendication 1 de l'acide 4-(2-oxo-3-bornylidènemethyl) benzène sulfonique ou de ses sels en des quantités comprises entre 0,4 et 9% en poids d'acide libre, dans un support aqueux, alcoolique ou hydroalcoolique, cosmétiquement acceptable.

4. Utilisation selon l'une quelconque des revendications 1 à 3, des sels alcalins, alcalino-terreux, d'ammonium ou d'amines de l'acide 4-(2-oxo-3-bornylidènemethyl) benzène sulfonique.

5. Utilisation selon l'une quelconque des revendications 1 à 4 de l'acide 4-(2-oxo-3-bornylidènemethyl)benzène sulfonique ou de ses sels dans des compositions cosmétiques pour cheveux se présentant sous forme de solutions aqueuses, alcooliques ou hydroalcooliques, épaissies ou non, de gels, de mousses aérosols ou de sprays.

6. Utilisation selon la revendication 5 de l'acide 4-(2-oxo-3-bornylidènemethyl) benzène sulfonique ou de ses sels dans des compositions cosmétiques pour cheveux contenant en outre au moins un adjuvant cosmétique choisi parmi les agents de surface cationiques, anioniques, amphotères, non-ioniques ou leurs mélanges, les épaississants, les polymères anioniques, cationiques, non-ioniques, amphotères ou leurs mélanges, les adoucissants, les conservateurs, les agents de moussage, les stabilisateurs de mousse, les électrolytes, les agents de régulation du pH, les agents anti-gras, les agents séquestrants, les parfums, les colorants, les propulseurs et les solvants organiques.

7. Utilisation selon l'une quelconque des revendications 1 à 6 de l'acide 4-(2-oxo-3-bornylidènemethyl) benzène sulfonique ou de ses sels dans des compositions cosmétiques rincées, aqueuses, alcooliques ou hydroalcooliques, en des quantités comprises entre 0,5 et 9% en poids, et de préférence entre 0,5 et 6% en poids d'acide libre, par rapport au poids total de la composition.

8. Utilisation selon l'une quelconque des revendications 1 à 6 de l'acide 4-(2-oxo-3-bornylidènemethyl)benzène sulfonique ou de ses sels dans des compositions cosmétiques non rincées, aqueuses, alcooliques ou hydroalcooliques, en des quantités comprises entre 0,3 et 5% en poids, et de préférence entre 0,4 et 3,5% en poids d'acide libre.

9. Utilisation selon l'une quelconque des revendications 1 à 7 de l'acide 4-(2-oxo-3-bornylidènemethyl) benzène sulfonique ou de ses sels dans des shampooings contenant, outre l'acide sulfonique ou ses sels, au moins un agent de surface anionique, non-ionique, cationique, amphotère ou leurs mélanges, dans des proportions comprises entre 2 et 50% en poids, en milieu aqueux.

10. Utilisation selon l'une quelconque des revendications 1 à 8 de l'acide 4-(2-oxo-3-bornylidènemethyl) benzène sulfonique ou de ses sels dans des compositions cosmétiques sous forme de mousses, à rincer ou non, contenant en milieu aqueux ou hydroalcoolique, en présence d'un gaz propulseur, un agent de moussage choisi parmi les tensio-actifs anioniques, non-ioniques, cationiques, amphotères ou leurs mélanges, les polymères non-ioniques, anioniques, cationiques ou leurs mélanges et l'alcool polyvinylique issu de polyacétate de vinyle hydrolysé dont le taux d'hydrolyse est égal ou inférieur à 97%.

11. Utilisation selon la revendication 10, de l'acide 4-(2-oxo-3-bornylidènemethyl) benzène sulfonique ou de ses sels, dans une composition cosmétique sous forme de mousse, à rincer ou non, contenant une association d'un polymère cationique et d'un polymère anionique en milieu aqueux ou hydroalcoolique, au moins l'un des deux polymères étant moussant en solution aqueuse, en présence d'un gaz propulseur.

12. Utilisation selon l'une quelconque des revendications 1 à 8 de l'acide 4-(2-oxo-3-bornylidènemethyl) benzène sulfonique ou de ses sels dans un spray, contenant dans un milieu alcoolique ou hydroalcoolique, éventuellement en présence d'un gaz propulseur, une résine filmogène constituée par un polymère anionique contenant des unités d'acides acrylique ou méthacrylique, d'acide crotonique ou d'acides α,β-dicarboxyliques insaturés.

13. Procédé de protection des propriétés mécaniques des cheveux contre les agressions atmosphériques, et en particulier contre la lumière, caractérisé par le fait qu'il consiste à appliquer sur les cheveux, une quantité efficace d'acide 4-(2-oxo-3-bornylidènemethyl) benzène sulfonique ou de ses sels, dans un support aqueux,

9

alcoolique ou hydroalcoolique, cosmétiquement acceptable.

14. Procédé selon la revendication 13, caractérisé par le fait qu'il consiste à appliquer sur les cheveux une composition contenant au moins 0,3% en poids d'acide 4-(2-oxo-3-bornylidèneméthyl) benzène sulfonique, dans un support aqueux, alcoolique ou hydroalcoolique, cosmétiquement acceptable.

15. Procédé selon la revendication 14, caractérisé par le fait qu'il consiste à appliquer sur les cheveux une composition contenant 0,4 à 9% en poids de l'acide 4-(2-oxo-3-bornylidèneméthyl) benzène sulfonique, dans un support aqueux, alcoolique ou hydroalcoolique, cosmétiquement acceptable.

16. Procédé selon l'une quelconque des revendications 13 à 15 caractérisé par le fait qu'il consiste à appliquer sur les cheveux une composition contenant un sel alcalin, alcalino-terreux, d'ammonium ou d'amine de l'acide 4-(2-oxo-3-bornylidèneméthyl) benzène sulfonique.

17. Procédé selon l'une quelconque des revendications 13 à 16, caractérisé par le fait qu'il consiste à appliquer sur les cheveux une composition cosmétique se présentant sous forme de solution aqueuse, alcoolique ou hydroalcoolique, épaissie ou non, de gel, de mousse aérosol ou de spray.

18. Procédé selon l'une quelconque des revendications 13 à 17, caractérisé par le fait qu'il consiste à appliquer sur les cheveux une composition cosmétique contenant en outre au moins un adjuvant cosmétique choisi parmi les agents de surface cationiques, anioniques, amphotères, non-ioniques ou leurs mélanges, les épaississants, les polymères anioniques, cationiques, non-ioniques, amphotères ou leurs mélanges, les adoucissants, les conservateurs, les agents de moussage, les stabilisateurs de mousse, les électrolytes, les agents de régulation du pH, les agents anti-gras, les agents séquestrants, les parfums, les colorants, les propulseurs et les solvants organiques.

19. Procédé selon l'une quelconque des revendications 13 à 18, caractérisé par le fait qu'il consiste à appliquer sur les cheveux une composition cosmétique rincée, aqueuse, alcoolique ou hydroalcoolique, contenant 0,5 et 9% en poids, et de préférence 0,5 et 6% en poids d'acide libre, par rapport au poids total de la composition.

20. Procédé selon l'une quelconque des revendications 13 à 18, caractérisé par le fait qu'il consiste à appliquer sur les cheveux une composition cosmétique non rincée, aqueuse, alcoolique ou hydroalcoolique, contenant 0,3 à 5% en poids, et de préférence 0,4 à 3,5% en poids d'acide libre.

21. Procédé selon l'une quelconque des revendications 13 à 19, caractérisé par le fait qu'il consiste à appliquer sur les cheveux, une composition sous forme de shampooing contenant, outre l'acide sulfonique ou ses sels, au moins un agent de surface anionique, non-ionique, cationique, amphotère ou leurs mélanges, dans des proportions comprises entre 2 et 50% en poids, en milieu aqueux.

22. Procédé selon l'une quelconque des revendications 13 à 20 caractérisé par le fait qu'il consiste à appliquer sur les cheveux une composition cosmétique sous forme de mousse, à rincer ou non, contenant en milieu aqueux, alcoolique ou hydroalcoolique, en présence d'un gaz propulseur, un agent de moussage choisi parmi les tensio-actifs anioniques, non-ioniques, cationiques, amphotères ou leurs mélanges, les polymères non-ioniques, anioniques, cationiques ou leurs mélanges et l'alcool polyvinylique issu de polyacétate de vinyle hydrolysé dont le taux d'hydrolyse est égal ou inférieur à 97%.

23. Procédé selon la revendication 22, caractérisé par le fait qu'il consiste à appliquer sur les cheveux une composition cosmétique sous forme de mousse, à rincer ou non, contenant une association d'un polymère cationique et d'un polymère anionique en milieu aqueux ou hydroalcoolique, au moins l'un des deux polymères étant moussant en solution aqueuse, en présence d'un gaz propulseur.

24. Procédé selon l'une quelconque des revendications 13 à 20, caractérisé par le fait qu'il consiste à appliquer sur les cheveux une composition cosmétique sous forme de spray, contenant dans un milieu alcoolique ou hydroalcoolique, éventuellement en présence d'un gaz propulseur, une résine filmgène constituée par un polymère anionique contenant des unités d'acides acrylique ou méthacrylique, d'acide crotonique ou d'acides α,β-dicarboxyliques insaturés.

**Patentansprüche**

**Patentansprüche für folgende Vertragsstaaten : AT, BE, CH, DE, FR, GB, GR, IT, LI, NL, SE**

1. Verwendung der 4-[(2-Oxo-3-bornyliden)methyl]benzolsulfonsäure oder von deren Salzen zur Bewahrung der mechanischen Eigenschaften der Haare vor einer Degradierung durch atmosphärische Einflüsse, und insbesondere durch Licht.

2. Verwendung gemäß Anspruch 1 der 4-[(2-Oxo-3-bornyliden)methyl]benzolsulfonsäure oder von deren Salzen in einer Menge von mindestens gleich 0,3 Gew.% an freier Säure in einem wäßrigen, alkoholischen oder wäßrig-alkoholischen, kosmetisch verträglichen Träger.

3. Verwendung gemäß Anspruch 1 der 4-[(2-Oxo-3-bornyliden)methyl]benzolsulfonsäure oder von deren Salzen in Mengen zwischen 0,4 und 9 Gew.% an freier Säure in einem wäßrigen, alkoholischen oder wäßrig-alkoholischen, kosmetisch verträglichen Träger.

4. Verwendung gemäß jedem der Ansprüche 1 bis 3 der Alkali-, Erdalkali-, Ammonium- oder Aminsalze der 4-[(2-Oxo-3-bornyliden)methyl]benzolsulfonsäure.

5. Verwendung gemäß jedem der Ansprüche 1 bis 4 der 4-[(2-Oxo-3-bornyliden)methyl]benzolsulfonsäure oder von deren Salzen in kosmetischen Zusammensetzungen für Haare, wobei die Zusammensetzungen in Form von wäßrigen, alkoholischen oder wäßrig-alkoholischen Lösungen, verdickt oder nicht, von Gelen, Aerosolschäumen oder Sprays vorliegen.

6. Verwendung gemäß Anspruch 5 der 4-[(2-Oxo-3-bornyliden)methyl]benzolsulfonsäure oder von deren Salzen in kosmetischen Zusammensetzungen für Haare, welche außerdem mindestens einen kosmetischen Hilfsstoff enthalten, ausgewählt unter kationischen, anionischen, amphoteren, nicht-ionischen Oberflächenmitteln oder deren Mischungen, Verdickungsmitteln, anionischen, kationischen, nicht-ionischen, amphoteren Polymeren oder deren Mischungen, Weichmachungsmitteln, Konservierungsmitteln, Schäumungsmitteln, Schaumstabilisatoren, Elektrolyten, pH-Regulatoren, Antifettmitteln, Sequestriermitteln, Parfüms, Färbemitteln, Treibmitteln und organischen Lösungsmitteln.

7. Verwendung gemäß jedem der Ansprüche 1 bis 6 der 4-[(2-Oxo-3-bornyliden)methyl]benzolsulfonsäure oder von deren Salzen in wäßrigen, alkoholischen oder wäßrig-alkoholischen kosmetischen Spülzusammensetzungen in Mengen zwischen 0,5 und 9 Gew.%, vorzugsweise zwischen 0,5 und 6 Gew.%, an freier Säure, bezogen auf das Gesamtgewicht der Zusammensetzung.

8. Verwendung gemäß jedem der Ansprüche 1 bis 6 der 4-[(2-Oxo-3-bornyliden)methyl]benzolsulfonsäure oder von deren Salzen in wäßrigen, alkoholischen oder wäßrig-alkoholischen kosmetischen Zusammensetzungen, die nicht gespült werden, in Mengen zwischen 0,3 und 5 Gew.%, vorzugsweise zwischen 0,4 und 3,5 Gew.%, an freier Säure.

9. Verwendung gemäß jedem der Ansprüche 1 bis 7 der 4-[(2-Oxo-3-bornyliden)methyl]benzolsulfonsäure oder von deren Salzen in Shampoons, enthaltend außer der Sulfonsäure oder deren Salzen mindestens ein anionisches, nicht-ionisches, kationisches, amphoteres Oberflächenmittel oder deren Mischungen in Mengen zwischen 2 und 50 Gew.% in wäßrigem Milieu.

10. Verwendung gemäß jedem der Ansprüche 1 bis 8 der 4-[(2-Oxo-3-bornyliden)methyl]benzolsulfonsäure oder von deren Salzen in kosmetischen Zusammensetzungen in Form von Schäumen, zur Spülung oder nicht zur Spülung, enthaltend in wäßrigem oder wäßrig-alkoholischem Milieu in Gegenwart eines Treibmittelgases ein Schäumungsmittel, ausgewählt aus anionischen, nicht-ionischen, kationischen, amphoteren oberflächenaktiven Mitteln oder deren Mischungen, nicht-ionischen, anionischen, kationischen Polymeren oder deren Mischungen und aus Polyvinylalkohol, hervorgegangen aus hydrolysiertem Polyvinylacetat mit einem Hydrolysegrad von 97% oder darunter.

11. Verwendung gemäß Anspruch 10 der 4-[(2-Oxo-3-bornyliden)methyl]benzolsulfonsäure oder von deren Salzen in einer kosmetischen Zusammensetzung in Form eines Schaums, zur Spülung oder nicht zur Spülung, enthaltend eine Verbindung eines kationischen und eines anionischen Polymers in wäßrigem oder wäßrig-alkoholischem Milieu, wobei mindestens eines der beiden Polymere in wäßriger Lösung schäumend ist, in Gegenwart eines Treibmittelgases.

12. Verwendung gemäß jedem der Ansprüche 1 bis 8 der 4-[(2-Oxo-3-bornyliden)methyl]benzolsulfonsäure oder von deren Salzen in einem Spray, enthaltend in alkoholischem oder wäßrig-alkoholischem Milieu, ggf. in Anwesenheit eines Treibmittelgases, ein filmbildendes Harz aus einem anionischen Polymer, enthaltend Einheiten der Acryl- oder Methacrylsäure, Crotonsäure oder von ungesättigten alpha,beta-Dicarbonsäuren.

13. Verfahren zum Schutz des Keratins der Haare gegen atmosphärische Einflüsse, und insbesondere gegen das Licht, dadurch gekennzeichnet, daß man auf die Haare eine wirksame Menge der 4-[(2-Oxo-3-bornyliden)methyl]benzolsulfonsäure oder von deren Salzen in einem wäßrigen, alkoholischen oder wäßrig-alkohlischen, kosmetisch verträglichen Träger aufbringt.

14. Verfahren gemäß Anspruch 13, dadurch gekennzeichnet, daß man auf die Haare eine Zusammensetzung aufbringt, enthaltend mindestens 0,3 Gew.% 4-[(2-Oxo-3-bornyliden)methyl]benzolsulfonsäure oder von deren Salzen in einem wäßrigen, alkoholischen oder wäßrig-alkohlischen, kosmetisch verträglichen Träger.

**Patentanspruch für folgenden Vertragsstaat : ES**

1. Verwendung der 4-[(2-Oxo-3-bornyliden)methyl]benzolsulfonsäure oder von deren Salzen zur Bewahrung der mechanischen Eigenschaften der Haare vor einer Degradierung durch atmosphärische Einflüsse, und insbesondere durch Licht.

2. Verwendung gemäß Anspruch 1 der 4-[(2-Oxo-3-bornyliden)methyl]benzolsulfonsäure oder von deren Salzen in einer Menge von mindestens gleich 0,3 Gew.% an freier Säure in einem wäßrigen, alkoholischen oder wäßrig-alkoholischen, kosmetisch verträglichen Träger.

3. Verwendung gemäß Anspruch 1 der 4-[(2-Oxo-3-bornyliden)methyl]benzolsulfonsäure oder von deren Salzen in Mengen zwischen 0,4 und 9 Gew.% an freier Säure in einem wäßrigen, alkoholischen oder wäßrig-alkoholischen, kosmetisch verträglichen Träger.

4. Verwendung gemäß jedem der Ansprüche 1 bis 3 der Alkali-, Erdalkali-, Ammonium- oder Aminsalze der 4-[(2-Oxo-3-bornyliden)methyl]benzolsulfonsäure.

5. Verwendung gemäß jedem der Ansprüche 1 bis 4 der 4-[(2-Oxo-3-bornyliden)methyl]benzolsulfonsäure oder von deren Salzen in kosmetischen Zusammensetzungen für Haare, wobei die Zusammensetzungen in Form von wäßrigen, alkoholischen oder wäßrig-alkoholischen Lösungen, verdickt oder nicht, von Gelen, Aerosolschäumen oder Sprays vorliegen.

6. Verwendung gemäß Anspruch 5 der 4-[(2-Oxo-3-bornyliden)methyl]benzolsulfonsäure oder von deren Salzen in kosmetischen Zusammensetzungen für Haare, welche außerdem mindestens einen kosmetischen Hilfsstoff enthalten, ausgewählt unter kationischen, anionischen, amphoteren, nicht-ionischen Oberflächenmitteln oder deren Mischungen, Verdickungsmitteln, anionischen, kationischen, nicht-ionischen, amphoteren Polymeren oder deren Mischungen, Weichmachungsmitteln, Konservierungsmitteln, Schäumungsmitteln, Schaumstabilisatoren, Elektrolyten, pH-Regulatoren, Antifettmitteln, Sequestriermitteln, Parfüms, Färbemitteln, Treibmitteln und organischen Lösungsmitteln.

7. Verwendung gemäß jedem der Ansprüche 1 bis 6 der 4-[(2-Oxo-3-bornyliden)methyl]benzolsulfonsäure oder von deren Salzen in wäßrigen, alkoholischen oder wäßrig-alkoholischen kosmetischen Spülzusammensetzungen in Mengen zwischen 0,5 und 9 Gew.%, vorzugsweise zwischen 0,5 und 6 Gew.%, an freier Säure, bezogen auf das Gesamtgewicht der Zusammensetzung.

8. Verwendung gemäß jedem der Ansprüche 1 bis 6 der 4-[(2-Oxo-3-bornyliden)methyl]benzolsulfonsäure oder von deren Salzen in wäßrigen, alkoholischen oder wäßrig-alkoholischen kosmetischen Zusammensetzungen, die nicht gespült werden, in Mengen zwischen 0,3 und 5 Gew.%, vorzugsweise zwischen 0,4 und 3,5 Gew.%, an freier Säure.

9. Verwendung gemäß jedem der Ansprüche 1 bis 7 der 4-[(2-Oxo-3-bornyliden)methyl]benzolsulfonsäure oder von deren Salzen in Shampoons, enthaltend außer der Sulfonsäure oder deren Salzen mindestens ein anionisches, nicht-ionisches, kationisches, amphoteres Oberflächenmittel oder deren Mischungen in Mengen zwischen 2 und 50 Gew.% in wäßrigem Milieu.

10. Verwendung gemäß jedem der Ansprüche 1 bis 8 der 4-[(2-Oxo-3-bornyliden)methyl]benzolsulfonsäure oder von deren Salzen in kosmetischen Zusammensetzungen in Form von Schäumen, zur Spülung oder nicht zur Spülung, enthaltend in wäßrigen oder wäßrig-alkoholischem Milieu in Gegenwart eines Treibmittelgases ein Schäumungsmittel, ausgewählt aus anionischen, nicht-ionischen, kationischen, amphoteren oberflächenaktiven Mitteln oder deren Mischungen, nicht-ionischen, anionischen, kationischen Polymeren oder deren Mischungen und aus Polyvinylalkohol, hervorgegangen aus hydrolysiertem Polyvinylacetat mit einem Hydrolysegrad von 97% oder darunter.

11. Verwendung gemäß Anspruch 10 der 4-[(2-Oxo-3-bornyliden)methyl]benzolsulfonsäure oder von deren Salzen in einer kosmetischen Zusammensetzung in Form eines Schaums, zur Spülung oder nicht zur Spülung, enthaltend eine Verbindung eines kationischen und eines anionischen Polymers in wäßrigem oder wäßrig-alkoholischem Milieu, wobei mindestens eines der beiden Polymere in wäßriger Lösung schäumend ist, in Gegenwart eines Treibmittelgases.

12. Verwendung gemäß jedem der Ansprüche 1 bis 8 der 4-[(2-Oxo-3-bornyliden)methyl]benzolsulfonsäure oder von deren Salzen in einem Spray, enthaltend in alkoholischem oder wäßrig-alkoholischem Milieu, ggf. in Anwesenheit eines Treibmittelgases, ein filmbildendes Harz aus einem anionischen Polymer, enthaltend Einheiten der Acryl- oder Methacrylsäure, Crotonsäure oder von ungesättigten alpha,beta-Dicarbonsäuren.

13. Verfahren zum Schutz der mechanischen Eigenschaften der Haare gegen atmosphärische Einflüsse, und insbesondere gegen das Licht, dadurch gekennzeichnet, daß man auf die Haare eine wirksame Menge der 4-[(2-Oxo-3-bornyliden)methyl]benzolsulfonsäure oder von deren Salzen in einem wäßrigen, alkoholischen oder wäßrig-alkoholischen, kosmetisch verträglichen Träger aufbringt.

14. Verfahren gemäß Anspruch 13, dadurch gekennzeichnet, daß man auf die Haare eine Zusammensetzung aufbringt, enthaltend mindestens 0,3 Gew.% 4-[(2-Oxo-3-bornyliden)methyl]benzolsulfonsäure oder von deren Salzen in einem wäßrigen, alkoholischen oder wäßrig-alkoholischen, kosmetisch verträglichen Träger.

15. Verfahren gemäß Anspruch 14, dadurch gekennzeichnet, daß man auf die Haare eine Zusammensetzung aufbringt, enthaltend 0,4 bis 9 Gew.% 4-[(2-Oxo-3-bornyliden)methyl]benzolsulfonsäure oder von deren Salzen in einem wäßrigen, alkoholischen oder wäßrig-alkoholischen, kosmetisch verträglichen Träger.

16. Verfahren gemäß jedem der Ansprüche 13 bis 15, dadurch gekennzeichnet, daß man auf die Haare eine Zusammensetzung aufbringt, enthaltend ein Alkali-, Erdalkali-, Ammonium- oder Aminsalz der 4-[(2-Oxo-3-bornyliden)methyl]benzolsulfonsäure.

17. Verfahren gemäß jedem der Ansprüche 13 bis 16, dadurch gekennzeichnet, daß man auf die Haare eine kosmetische Zusammensetzung aufbringt, die in Form einer wäßrigen, alkoholischen oder wäßrig-alkoholischen Lösung, verdickt oder nicht, eines Gels, eines Aerosolschaums oder eines Sprays vorliegt.

18. Verfahren gemäß jedem der Ansprüche 13 bis 17, dadurch gekennzeichnet, daß man auf die Haare eine kosmetische Zusammensetzung aufbringt, enthaltend außerdem mindestens einen kosmetischen Hilfsstoff, ausgewählt aus kationischen, anionischen, amphoteren, nicht-ionischen Oberflächenmitteln oder deren Mischungen, Verdickungsmitteln, anionischen, kationischen, nicht-ionischen, amphoteren Polymeren oder deren Mischungen, Weichmachungsmitteln, Konservierungsmitteln, Schäumungsmitteln, Schaumstabilisatoren, Elektrolyten, pH-Regulatoren, Antifettmitteln, Sequestriermitteln, Parfüms, Färbemitteln, Treibmitteln und organischen Lösungsmitteln.

19. Verfahren gemäß jedem der Ansprüche 13 bis 18, dadurch gekennzeichnet, daß man auf die Haare eine wäßrige, alkoholische oder wäßrig-alkoholische, kosmetische Spülzusammensetzung aufbringt, enthaltend 0,5 bis 9 Gew.%, vorzugsweise 0,5 bis 6 Gew.% an freier Säure, bezogen auf das Gesamtgewicht der Zusammensetzung.

20. Verfahren gemäß jedem der Ansprüche 13 bis 18, dadurch gekennzeichnet, daß man auf die Haare eine wäßrige, alkoholische oder wäßrig-alkoholische, kosmetische Zusammensetzung, die nicht gespült wird, aufbringt, enthaltend 0,3 bis 5 Gew.%, vorzugsweise 0,4 bis 3,5 Gew.%, an freier Säure.

21. Verfahren gemäß jedem der Ansprüche 13 bis 19, dadurch gekennzeichnet, daß man auf die Haare eine Zusammensetzung in Form eines Shampoons aufbringt, enthaltend außer der Sulfonsäure oder deren Salzen mindestens ein anionisches, nicht-ionisches, kationisches, amphoteres Oberflächenmittel oder deren Mischungen in Mengen zwischen 2 und 50 Gew.% in wäßrigem Milieu.

22. Verfahren gemäß jedem der Ansprüche 13 bis 20, dadurch gekennzeichnet, daß man auf die Haare eine kosmetische Zusammensetzung in Form eines Schaums, zur Spülung oder nicht zur Spülung, aufbringt, enthaltend in wäßrigem, alkoholischem oder wäßrig-alkoholischem Milieu in Gegenwart eines Treibmittelgases ein Schäumungsmittel, ausgewählt aus anionischen, nicht-ionischen, kationischen, amphoteren oberflächenaktiven Mitteln oder deren Mischungen, nicht-ionischen, anionischen, kationischen Polymeren oder deren Mischungen und aus Polyvinylalkohol, hervorgegangen aus hydrolysiertem Polyvinylacetat mit einem Hydrolysegrad von 97% oder darunter.

23. Verfahren gemäß Anspruch 22, dadurch gekennzeichnet, daß man auf die Haare eine kosmetische Zusammensetzung in Form eines Schaums, zur Spülung oder nicht zur Spülung, aufbringt, enthaltend eine Verbindung eines kationischen und anionischen Polymers in wäßrigem oder wäßrig-alkoholischem Medium, wobei mindestens eines der beiden Polymere in wäßriger Lösung schäumend ist, in Gegenwart eines Treibmittelgases.

24. Verfahren gemäß jedem der Ansprüche 13 bis 20, dadurch gekennzeichnet, daß man auf die Haare eine kosmetische Zusammensetzung in Form eines Sprays aufbringt, enthaltend in wäßrigem oder wäßrig-alkoholischem Milieu, ggf. in Gegenwart eines Treibmittelgases, ein filmbildendes Harz aus einem anionischen Polymer, enthaltend Einheiten der Acryl- oder Methylacrylsäure, der Crotonsäure oder von ungesättigten alpha,beta-Dicarbonsäuren.

## Claims

### Claims for the following Contracting States : AT, BE, CH, DE, FR, GB, GR, IT, LI, NL, SE

1. Use of 4-(2-oxo-3-bornylidenemethyl)benzene-sulphonic acid or its salts for preserving the mechanical properties of the hair from degradation by damaging environmental agents, and especially by light.

2. Use according to Claim 1 of 4-(2-oxo-3-bornylidenemethyl)benzenesulphonic acid or its salts in a quantity equal to at least 0.3% by weight of free acid, in a cosmetically acceptable, aqueous, alcoholic or aqueous-alcoholic vehicle.

3. Use according to Claim 1 of 4-(2-oxo-3-bornylidenemethyl)benzenesulphonic acid or its salts in quantities of between 0.4 and 9% by weight of free acid, in a cosmetically acceptable, aqueous, alcoholic or aqueous-alcoholic vehicle.

4. Use according to any one of Claims 1 to 3 of the alkali metal, alkaline earth metal, ammonium or amine salts of 4-(2-oxo-3-bornylidenemethyl)benzenesulphonic acid.

5. Use according to any one of Claims 1 to 4 of 4-(2-oxo-3-bornylidenemethyl) benzenesulphonic acid or its salts in cosmetic compositions for hair which take the form of aqueous, alcoholic or aqueous-alcoholic solutions, thickened or otherwise, gels, aerosol foams or sprays.

6. Use according to Claim 5 of 4-(2-oxo-3-bornylidenemethyl)benzenesulphonic acid or its salts in cosmetic compositions for hair, containing, in addition, at least one cosmetic adjuvant selected from cationic, anionic, amphoteric and nonionic surfactants or mixtures thereof, thickeners, anionic, cationic, nonionic and amphoteric polymers or mixtures thereof, emollients, preservatives, foaming agents, foam stabilisers, electrolytes, pH regulators, antigrease agents, sequestering agents, fragrances, colourings, propellants and organic solvents.

7. Use according to any one of Claims 1 to 6 of 4-(2-oxo-3-bornylidenemethyl)benzenesulphonic acid or its salts in rinsed aqueous, alcoholic or aqueous-alcoholic cosmetic compositions, in quantities of between 0.5 and 9% by weight, and preferably between 0.5 and 6% by weight, of free acid relative to the total weight of the composition.

8. Use according to any one of Claims 1 to 6 of 4-(2-oxo-3-bornylidenemethyl)benzenesulphonic acid or its salts in non-rinsed aqueous, alcoholic or aqueous-alcoholic cosmetic compositions, in quantities of between 0.3 and 5% by weight, and preferably between 0.4 and 3.5% by weight, of free acid.

9. Use according to any one of Claims 1 to 7 of 4-(2-oxo-3-bornylidenemethyl)benzenesulphonic acid or its salts in shampoos containing, apart from the sulphonic acid or its salts, at least one anionic, nonionic, cationic or amphoteric surfactant or mixtures thereof, in proportions of between 2 and 50% by weight, in an aqueous medium.

10. Use according to any one of Claims 1 to 8 of 4-(2-oxo-3-bornylidenemethyl)benzenesulphonic acid or its salts in cosmetic compositions in the form of foams, to be rinsed or otherwise, containing, in an aqueous or aqueous-alcoholic medium and in the presence of a propellent gas, a foaming agent selected from anionic, nonionic, cationic and amphoteric surfactants or mixtures thereof, nonionic, anionic and cationic polymers or mixtures thereof and polyvinyl alcohol derived from hydrolysed polyvinyl acetate having a degree of hydrolysis not exceeding 97%.

11. Use according to Claim 10 of 4-(2-oxo-3-bornylidenemethyl)benzenesulphonic acid or its salts in a cosmetic composition in the form of a foam, to be rinsed or otherwise, containing a combination of a cationic polymer and an anionic polymer in an aqueous or aqueous-alcoholic medium, at least one of the two polymers being foaming in aqueous solution, in the presence of a propellent gas.

12. Use according to any one of Claims 1 to 8 of 4-(2-oxo-3-bornylidenemethyl)benzenesulphonic acid or its salts in a spray containing, in an alcoholic or aqueous-alcoholic medium and optionally in the presence of a propellent gas, a film-forming resin consisting of an anionic polymer containing units of acrylic or methacrylic acid, of crotonic acid or of unsaturated $\alpha,\beta$-dicarboxylic acids.

13. Process for protecting the keratin of the hair against damaging environmental agents, and especially against light, characterised in that it consists in applying on the hair an effective quantity of 4-(2-oxo-3-bornylidenemethyl)benzenesulphonic acid or its salts, in a cosmetically acceptable, aqueous, alcoholic or aqueous-alcoholic vehicle.

14. Process according to Claim 13, characterised in that it consists in applying on the hair a composition containing at least 0.3% by weight of 4-(2-oxo-3-bornylidenemethyl)benzenesulphonic acid, in a cosmetically acceptable, aqueous, alcoholic or aqueous-alcoholic vehicle.

## Claims for the following Contracting State : ES

1. Use of 4-(2-oxo-3-bornylidenemethyl)benzenesulphonic acid or its salts for preserving the mechanical properties of the hair from degradation by damaging environmental agents, and especially by light.

2. Use according to Claim 1 of 4-(2-oxo-3-bornylidenemethyl)benzenesulphonic acid or its salts in a quantity equal to at least 0.3% by weight of free acid, in a cosmetically acceptable, aqueous, alcoholic or aqueous-alcoholic vehicle.

3. Use according to Claim 1 of 4-(2-oxo-3-bornylidenemethyl)benzenesulphonic acid or its salts in quantities of between 0.4 and 9% by weight of free acid, in a cosmetically acceptable, aqueous, alcoholic or aqueous-alcoholic vehicle.

4. Use according to any one of Claims 1 to 3 of the alkali metal, alkaline earth metal, ammonium or amine salts of 4-(2-oxo-3-bornylidenemethyl)benzenesulphonic acid.

5. Use according to any one of Claims 1 to 4 of 4-(2-oxo-3-bornylidenemethyl)benzenesulphonic acid or its salts in cosmetic compositions for hair which take the form of aqueous, alcoholic or aqueous-alcoholic solutions, thickened or otherwise, gels, aerosol foams or sprays.

6. Use according to Claim 5 of 4-(2-oxo-3-bornylidenemethyl)benzenesulphonic acid or its salts in cosmetic compositions for hair containing, in addition, at least one cosmetic adjuvant selected from cationic, anionic, amphoteric and nonionic surfactants or mixtures thereof, thickeners, anionic, cationic, nonionic and amphoteric polymers or mixtures thereof, emollients, preservatives, foaming agents, foam stabilisers, electrolytes, pH regulators, antigrease agents, sequestering agents, fragrances, colourings, propellants and organic solvents.

7. Use according to any one of Claims 1 to 6 of 4-(2-oxo-3-bornylidenemethyl)benzenesulphonic acid or its salts in rinsed aqueous, alcoholic or aqueous-alcoholic cosmetic compositions, in quantities of between 0.5 and 9% by weight, and preferably between 0.5 and 6% by weight, of free acid relative to the total weight of the composition.

8. Use according to any one of Claims 1 to 6 of 4-(2-oxo-3-bornylidenemethyl)benzenesulphonic acid or its salts in non-rinsed aqueous, alcoholic or aqueous-alcoholic cosmetic compositions, in quantities of between 0.3 and 5% by weight, and preferably between 0.4 and 3.5% by weight, of free acid.

9. Use according to any one of Claims 1 to 7 of 4-(2-oxo-3-bornylidenemethyl)benzenesulphonic acid or its salts in shampoos containing, apart from the sulphonic acid or its salts, at least one anionic, nonionic, cationic or amphoteric surfactant or mixtures thereof, in proportions of between 2 and 50% by weight, in an aqueous medium.

10. Use according to any one of Claims 1 to 8 of 4-(2-oxo-3-bornylidenemethyl)benzenesulphonic acid or its salts in cosmetic compositions in the form of foams, to be rinsed or otherwise, containing, in an aqueous or aqueous-alcoholic medium and in the presence of a propellent gas, a foaming agent selected from anionic, nonionic, cationic and amphoteric surfactants or mixtures thereof, nonionic, anionic and cationic polymers or mixtures thereof and polyvinyl alcohol derived from hydrolysed polyvinyl acetate having a degree of hydrolysis not exceeding 97%.

11. Use according to Claim 10 of 4-(2-oxo-3-bornylidenemethyl)benzenesulphonic acid or its salts in a cosmetic composition in the form of a foam, to be rinsed or otherwise, containing a combination of a cationic polymer and an anionic polymer in an aqueous or aqueous-alcoholic medium, at least one of the two polymers being foaming in aqueous solution, in the presence of a propellent gas.

12. Use according to any one of Claims 1 to 8 of 4-(2-oxo-3-bornylidenemethyl)benzenesulphonic acid or its salts in a spray containing, in an alcoholic or aqueous-alcoholic medium and optionally in the presence of a propellent gas, a film-forming resin consisting of an anionic polymer containing units of acrylic or methacrylic acid, of crotonic acid or of unsaturated $\alpha,\beta$-dicarboxylic acids.

13. Process for protecting the mechanical properties of the hair against damaging environmental agents, and especially against light, characterised in that it consists in applying on the hair an effective quantity of 4-(2-oxo-3-bornylidenemethyl)benzenesulphonic acid or its salts, in a cosmetically acceptable, aqueous, alcoholic or aqueous-alcoholic vehicle.

14. Process according to Claim 13, characterised in that it consists in applying on the hair a composition containing at least 0.3% by weight of 4-(2-oxo-3-bornylidenemethyl)benzenesulphonic acid, in a cosmetically acceptable, aqueous, alcoholic or aqueous-alcoholic vehicle.

15. Process according to Claim 14, characterised in that it consists in applying on the hair a composition containing 0.4 to 9% by weight of 4-(2-oxo-3-bornylidenemethyl)benzenesulphonic acid, in a cosmetically acceptable, aqueous, alcoholic or aqueous-alcoholic vehicle.

16. Process according to any one of Claims 13 to 15, characterised in that it consists in applying on the hair a composition containing an alkali metal salt, alkaline earth metal salt, ammonium salt or amine salt of 4-(2-oxo-3-bornylidenemethyl)benzenesulphonic acid.

17. Process according to any one of Claims 13 to 16, characterised in that it consists in applying on the hair a cosmetic composition which takes the form of an aqueous, alcoholic or aqueous-alcoholic solution, thickened or otherwise, gel, aerosol foam or spray.

18. Process according to any one of Claims 13 to 17, characterised in that it consists in applying on the hair a cosmetic composition containing, in addition, at least one cosmetic adjuvant selected from cationic, anionic, amphoteric and nonionic surfactants or mixtures thereof, thickeners, anionic, cationic, nonionic and amphoteric polymers or mixtures thereof, emollients, preservatives, foaming agents, foam stabilisers, electrolytes, pH regulators, antigrease agents, sequestering agents, fragrances, colourings, propellants and organic solvents.

19. Process according to any one of Claims 13 to 18, characterised in that it consists in applying on the hair a rinsed aqueous, alcoholic or aqueous-alcoholic cosmetic composition containing 0.5 to 9% by weight, and preferably 0.5 to 6% by weight, of free acid relative to the total weight of the composition.

20. Process according to any one of Claims 13 to 18, characterised in that it consists in applying on the hair a non-rinsed aqueous, alcoholic or aqueous-alcoholic cosmetic composition containing 0.3 to 5% by weight, and preferably 0.4 to 3.5% by weight, of free acid.

21. Process according to any one of Claims 13 to 19, characterised in that it consists in applying on the hair a composition in the form of a shampoo containing, apart from the sulphonic acid or its salts, at least one anionic, nonionic, cationic or amphoteric surfactant or mixtures thereof, in proportions of between 2 and 50% by weight, in an aqueous medium.

22. Process according to any one of Claims 13 to 20, characterised in that it consists in applying on the hair a cosmetic composition in the form of a foam, to be rinsed or otherwise, containing, in an aqueous, alcoholic or aqueous-alcoholic medium and in the presence of a propellent gas, a foaming agent selected from anionic, nonionic, cationic and amphoteric surfactants or mixtures thereof, nonionic, anionic and cationic polymers or mixtures thereof and polyvinyl alcohol derived from hydrolysed polyvinyl acetate having a degree of hydrolysis not exceeding 97%.

23. Process according to Claim 22, characterised in that it consists in applying on the hair a cosmetic composition in the form of a foam, to be rinsed or otherwise, containing a combination of a cationic polymer and an anionic polymer in an aqueous or aqueous-alcoholic medium, at least one of the two polymers being foaming in aqueous solution, in the presence of a propellent gas.

24. Process according to any one of Claims 13 to 20, characterised in that it consists in applying on the hair a cosmetic composition in the form of a spray containing, in an alcoholic or aqueous-alcoholic medium and optionally in the presence of a propellent gas, a film-forming resin consisting of an anionic polymer containing units of acrylic or methacrylic acid, of crotonic acid or of unsaturated $\alpha,\beta$-dicarboxylic acids.